# FASCICULE DE BREVET EUROPEEN

(11) **EP 1 071 657 B1**
(45) Date de publication et mention de la délivrance du brevet: **20.08.2003**
(21) Numéro de dépôt: 99913398.6
(22) Date de dépôt: 13.04.1999
(51) Int. Cl.: C07C 253/34, C07C 255/58

(54) **PROCEDE DE PURIFICATION D'AMINONITRILES ALIPHATIQUES**
VERFAHREN ZUR REINIGUNG VON AMINONITRILEN
METHOD FOR PURIFYING ALIPHATIC AMINONITRILES

(30) Priorité: 16.04.1998 FR 9805044
(43) Date de publication de la demande: 31.01.2001
(73) Titulaire: Rhodia Polyamide Intermediates, 69192 Saint-Fons (FR)
(72) Inventeur: BRUNELLE, Jean-Pierre, F-78290 Croissy-sur-Seine (FR); LECONTE, Philippe, F-69330 Meyzieu (FR); MARION, Philippe, F-69390 Vernaison (FR)
(74) Mandataire: Esson, Jean-Pierre
(86) Numéro de dépôt international: FR9900862
(87) Numéro de publication internationale: WO99054285

(56) Documents cités:
- EP-A- 0 502 439
- WO-A-93/14064
- WO-A-98/34899

## Description

La présente invention concerne un procédé de purification d'aminonitriles aliphatiques et plus particulièrement de l'amino-6 capronitrile.

L'amino-6 capronitrile est préparé généralement par hydrogénation d'une des deux fonctions nitrile de l'adiponitrile. Ainsi le brevet US-A-5 151 543 décrit un procédé d'hydrogénation partielle de dinitriles en aminonitriles dans un solvant en excès molaire d'au moins 2/1 par rapport au dinitrile, comprenant de l'ammoniac liquide ou un alcanol contenant une base minérale soluble dans ledit alcanol, en présence d'un catalyseur de type nickel ou cobalt de Raney.

Le brevet WO-A-96/18603 décrit un procédé d'hémihydrogénation de dinitriles aliphatiques en aminonitriles correspondants, à l'aide d'hydrogène et en présence d'un catalyseur choisi parmi le nickel de Raney et le cobalt de Raney, ledit nickel ou cobalt de Raney comportant éventuellement un élément dopant choisi parmi les éléments des groupes IVb, Vlb, Vllb et VIII de la classifrcation périodique des éléments et le zinc, et d'une base minérale forte dérivant d'un métal alcalin ou alcalino-terreux, le milieu initial d'hydrogénation comportant de l'eau à raison d'au moins 0,5 % en poids par rapport à la totalité des composés liquides dudit milieu, de la diamine et/ou de l'aminonitrile susceptibles de se former à partir du dinitrile à hydrogéner ainsi que du dinitrile non transformé à raison pour l'ensemble de ces trois composés de 80 % à 99,5 % en poids par rapport à la totalité des composés liquides dudit milieu, ledit procédé permettant d'obtenir une sélectivité en aminonitriles visés d'au moins 60 %.

Une des utilisations possibles de l'amino-6 capronitrile (aussi appelé ACN par commodité dans le présent texte) consiste à le faire réagir avec l'eau et à le cycliser (hydrolyse cyclisante) pour obtenir le caprolactame, qui est la matière première du polyamide 6.

Cette hydrolyse cyclisante peut être réalisée en phase liquide comme décrit dans le brevet WO-A-96/00722 ou en phase vapeur comme décrit dans le brevet EP-A-0 659 741 ou le brevet WO-A-96/22974.

L'amino-6 capronitrile soumis à l'hydrolyse cyclisante peut contenir jusqu'à quelques pourcents d'impuretés, telles que l'hexaméthylènediamine ou diverses imines formées lors de l'hydrogénation de l'adiponitrile, sans que cela soit rédhibitoire pour ladite réaction d'hydrolyse cyclisante. C'est notamment le cas lorsque l'hydrolyse est conduite en phase vapeur. Cependant une partie de ces impuretés, transformées ou non au cours de l'hydrolyse, peut se retrouver dans le caprolactame obtenu. Or même si les quantités d'impuretés susceptibles de se trouver dans le caprolactame sont relativement faibles, par exemple de l'ordre de 1 à 2 % , la pureté exigée lors de la polymérisation ultérieure du caprolactame est telle que la purification dudit caprolactame peut s'avérer complexe et très coûteuse. La purification de l'amino-6 capronitrile permet de simplifier les traitements de purification du caprolactame obtenu. Ainsi par exemple, le traitement du caprolactame décrit dans le brevet WO-A-98/05636 peut être effectué par passage sur résine échangeuse d'ions et distillation lorsque l'amino-6 capronitrile est purifié et peut comporter en outre une opération d'hydrogénation si l'amino-6 capronitrile n'a pas été suffisamment purifié avant son hydrolyse.

Une purification de l'amino-6 capronitrile avant sa transformation en caprolactame peut par conséquent être considérée comme techniquement et économiquement très utile.

La présente invention consiste en un procédé de purification relativement simple d'aminonitrile tel que la purification de l'amino-6 capronitrile obtenu après séparation de l'adiponitrile non transformé ou directement par traitement du milieu réactionnel d'hémihydrogénation de l'adiponitrile.

L'hexaméthylènediamine est coproduite avec l'amino-6 capronitrile lors de l'hydrogénation de l'adiponitrile. Elle peut soit être séparée, totalement ou en partie, dudit amino-6 capronitrile avant le traitement de celui-ci, soit de préférence être maintenue en mélange pour ledit traitement. Dans le présent texte, sauf précision particulière, le terme amino-6 capronitrile (ou ACN) couvrira donc l'amino-6 capronitrile et ses mélanges avec l'hexaméthylènediamine. Plus généralement, le terme aminonitrile désigne dans le présent texte un milieu contenant un aminonitrile à purifier et éventuellement une ou des diamines.

Elle consiste plus précisément à soumettre un aminonitrile et de préférence l'amino-6 capronitrile à une hydrogénation par l'hydrogène moléculaire, en présence d'un catalyseur contenant au moins un métal choisi parmi le palladium, le platine, le ruthénium, l'osmium, l'iridium, le rhodium.

Le procédé de l'invention s'applique plus particulièrement à la purification de l'amino-6-capronitrile et notamment de celui obtenu par hémihydrogénation de l'adiponitrile, tel que cela est décrit au paragraphe ci-dessus.

Ce catalyseur comporte soit un ou plusieurs éléments promoteurs choisis dans le groupe comportant l'or, l'argent, le cuivre, le chrome, le molybdène, le tungstène, le germanium, l'étain, le plomb, le bore, le gallium, l'indium, le thallium, le phosphore, l'arsenic, l'antimoine, le bismuth, le soufre, le sélénium, le tellure, le manganèse, le rhénium, le vanadium, le titane, le zinc, soit est soumis à une étape de préconditionnement consistant en une mise en contact avec un agent sélectivant avant ou en début de réaction d'hydrogénation.

Cette étape de préconditionnement est facultative quand le catalyseur comprend comme élément catalytique un élément choisi dans le groupe consistant en le ruthénium, l'osmium, l'iridium, le rhodium, en présence ou non d'éléments promoteur.

Au contraire, cette étape est nécessaire quand le catalyseur comprend un élément choisi dans le groupe comprenant le palladium, le platine, en absence d'éléments promoteur.

Ces éléments promoteurs peuvent être sous forme libre ou sous forme combinée, par exemple sous forme d'oxydes ou de sels. Ils ont pour but d'améliorer la sélectivité de l'hydrogénation des impuretés notamment des imines, en inhibant encore plus l'hydrogénation de la fonction nitrile de l'aminonitrile et notamment de l'ACN. Ils sont mis en oeuvre de préférence avec les catalyseurs au palladium, au platine et au ruthénium. Le rapport pondéral élément promoteur/métal catalyseur varie selon le promoteur. Il se situe généralement entre 0 % et 100 %. De préférence, ce rapport est compris entre 5 et 60 % en poids pour certains promoteurs comme l'argent. Pour d'autres promoteurs ce rapport pondéral varie entre 0,0001 % et 10 %.

Selon une autre caractéristique de l'invention, le catalyseur de l'invention peut être soumis ou est soumis, selon sa composition, à une étape de préconditionnement qui permet d'améliorer la sélectivité de l'hydrogénation, c'est à dire d'améliorer le taux d'hydrogénation des impuretés de l'aminonitrile telles que les imines tout en maintenant à une valeur minimale le taux d'hydrogénation de l'aminonitrile.

Cette étape de préconditionnement consiste à soumettre le catalyseur à un fluide contenant un agent sélectivant. Ce traitement peut être effectué sur le catalyseur avant son engagement dans le réacteur d'hydrogénation par traitement avec un flux d'hydrogène contenant une certaine quantité d'agent sélectivant.

Toutefois, dans un mode de réalisation préféré de l'invention, le préconditionnement du catalyseur est mis en oeuvre directement dans le réacteur d'hydrogénation, en début d'hydrogénation soit par alimentation d'un flux d'hydrogène contenant ledit agent sélectivant pendant une durée déterminée et suffisante pour alimenter la quantité désirée d'agent sélectivant, soit par alimentation dudit agent sélectivant en mélange avec le flux d'aminonitrile à hydrogéner pendant une durée correspondante à l'alimentation de la quantité désirée dudit agent.

Bien entendu, un agent sélectivant peut être présent simultanément dans le flux d'hydrogène et l'aminonitrile à traiter, cet agent pouvant être identique ou différent.

A titre d'exemple, on peut citer comme agent sélectivant convenable pour l'invention ceux appartenant au groupe comprenant l'oxyde de carbone, les composés organiques soufrés, les composés organiques phosphorés.

Plus précisément, les composés organiques soufrés peuvent être choisis dans le groupe comprenant la thiourée et ses dérivés, les thiols, les sulfures d'alkyles, et les composés organiques phosphorés dans le groupe comprenant les phosphites, hypophosphites d'alkyles, phosphates et thiophosphates d'alkyles.

Selon une autre caractéristique de l'invention, la quantité d'agent sélectivant ajouté par tonne de catalyseur à préconditionner (support inclus) est comprise avantageusement entre 0,5 et 15 mole d'agent sélectivant par tonne de catalyseur.

Le procédé d'hydrogénation de l'invention permet de diminuer très notablement la quantité d'impuretés notamment de type imine présentes dans l'aminonitrile et notamment dans l'amino-6-capronitrile. La présence de telles impuretés est illustrée et exprimée par un indice polarographique élevé (cet indice est dénommé généralement par le sigle IPOL).La présence de telles impuretés ou la mesure d'un IPOL élevé se traduirait en particulier par l'apparition d'une coloration et la formation de ramifications lors de la polymérisation du caprolactame obtenu par cyclisation de l'aminonitrile, si elles se retrouvaient dans ce dernier. Parmi ces imines, la tétrahydroazépine (THA) est l'une des plus connues et l'une des plus difficiles à éliminer, en particulier par distillation. Par contre l'hydrogénation mise en oeuvre selon le procédé de l'invention, n'affecte pas de manière importante la fonction nitrile de l'amino-6 capronitrile.

L'indice polarographique déterminé par polarographie est exprimé en moles de fonction imine par tonne d'échantillon à doser.

Il a été proposé différentes techniques d'élimination de la THA. Ainsi le brevet WO-A-93/14064 décrit la réaction de la THA avec un composé à groupe méthylène actif tel que le malonitrile ou le nitrométhane, afin d'obtenir un composé d'addition de masse moléculaire plus élevée pouvant être séparé plus facilement de l'amino-6 capronitrile. Une telle technique implique l'introduction d'un nouveau réactif, ce qui complique encore les traitements déjà complexes de l'amino-6 capronitrile. En outre, les composés utilisés sont relativement coûteux et peuvent pour certains être d'une utilisation délicate (nitrométhane ou nitroéthane par exemple).

Le brevet EP-A-0 502 439 décrit la purification de l'amino-6 capronitrile par réduction de la tétrahydroazépine à l'aide d'un hydrure, plus particulièrement de borohydrure de sodium en quantité largement supérieure à la quantité stoechiométrique, de préférence 4 à 5 fois ladite quantité stoechiométrique. Un tel procédé n'est guère envisageable sur un plan industriel en raison du coût très élevé de tels hydrures.

En outre la présence d'hydrure est susceptible de compliquer le traitement ultérieur de l'amino-6 capronitrile.

L'hydrogénation est une opération simple qui s'avère très efficace. Elle peut être conduite selon les techniques habituelles. On peut notamment opérer avec un catalyseur déposé en lit fixe, avec lequel l'ACN à traiter et l'hydrogène sont mis en contact.

On peut également réaliser l'hydrogénation avec un catalyseur mis en suspension . dans le milieu réactionnel à l'aide d'une agitation.

Le catalyseur mis en oeuvre comprend généralement le métal actif finement divisé et déposé sur un support solide. Comme support, on peut citer non limitativement des oxydes tels que l'alumine, la zircone, le dioxyde de titane, la silice ou encore les charbons actifs.

Parmi les catalyseurs mis en oeuvre dans le présent procédé, on préfère ceux qui contiennent au moins du palladium, qui s'avèrent particulièrement efficaces.

L'hydrogénation est réalisée à une température compatible avec la stabilité de l'aminonitrile et plus particulièrement de l'ACN, d'une part et permettant d'avoir une cinétique suffisante, d'autre part.

Une température comprise entre 20°C et 150°C convient habituellement. De préférence l'hydrogénation sera conduite à une température la plus faible possible et compatible avec l'objectif de cinétique.

La pression absolue d'hydrogène à la température d'hydrogénation'peut varier dans de larges limites. Elle se situe généralement entre 1 bar et 100 bar, mais est de préférence comprise entre 2 et 50 bar.

Il n'est pas nécessaire de diluer l'aminonitrile, par exemple l'ACN, dans un solvant pour le soumettre à l'opération d'hydrogénation. Cependant la présente invention peut être réalisée en présence de quantités variables de solvant. Le solvant susceptible d'être utilisé doit alors être inerte vis-à-vis de l'aminonitrile (l'ACN) et de l'hydrogène.
A titre d'exemple non limitatif de tels solvants, on peut citer l'eau et l'ammoniac.
La quantité d'eau peut varier dans de grandes proportions. Elle représente de préférence de 0 % à 40 % en poids du poids d'ACN à traiter et plus préférentiellement de 0 % à 30 %. L'ammoniac peut être utilisé en quantités encore plus variables, par exemple entre 0 % et 100 % en poids du poids d'ACN. La présence d'eau et d'ammoniac est aussi envisageable dans le procédé de l'invention.

L'hydrogénation est suivie de manière avantageuse par une distillation permettant de séparer l'ACN des impuretés hydrogénées, notamment des imines transformées en amines, ainsi que de l'hexaméthylènediamine qu'il peut contenir. Cette distillation est réalisée selon les techniques habituelles.

L'ACN purifié est ensuite soumis à une hydrolyse cyclisante conduisant à la formation de caprolactame, par réaction avec l'eau. Cette hydrolyse cyclisante est effectuée de manière connue, soit en phase vapeur, soit en phase liquide.

Les exemples donnés ci-dessous uniquement à titre indicatif illustrent l'invention.

### EXEMPLE 1 COMPARATIF

Dans un réacteur, on place en lit fixe 80 g d'un catalyseur constitué de Pd métallique déposé sur un support alumine (0,3 % en poids de Pd).

La réaction est réalisée à 80°C et sous une pression d'hydrogène (à chaud) de 20 bar.

L'amino-6 capronitrile mis en oeuvre présente un indice polarographique (IPOL) initial de 84 mole/t. On introduit 350 g de cet ACN, l'hydrogénation étant réalisée en 2 h 30 sur le lit catalytique.

On détermine la variation de l'indice IPOL et le taux de transformation de l'aminonitrile ou plus précisément la perte en aminonitrile due à l'hydrogénation de celui-ci. Cette dernière valeur est déterminée par analyse chromatographique en phase vapeur.

Les résultats obtenus sont rassemblés dans le tableau I ci-dessous.

### EXEMPLE 2 COMPARATIF

Dans un réacteur, on place en lit fixe 99 g d'un catalyseur constitué de Pd métallique déposé sur un support charbon actif (0,5 % en poids de Pd).

La réaction est réalisée à 80°C et sous une pression d'hydrogène (à chaud) de 20 bar.

L'amino-6 capronitrile mis en oeuvre présente un indice polarographique (IPOL) initial de 96 mole/t On introduit 278 g de cet ACN en 3 h sur le lit catalytique.

Les résultats obtenus sont rassemblés dans le tableau I ci-dessous.

### EXEMPLE 3 COMPARATIF

Dans un réacteur, on place en lit fixe 140 g d'un catalyseur constitué de Ni métallique déposé sur un support silice (56 % en poids de Ni).

La réaction est réalisée à 80°C et sous une pression d'hydrogène (à chaud) de 20 bar.

L'amino-6 capronitrile mis en oeuvre présente un indice polarographique (IPOL) initial de 92 mole/t. On introduit 299 g de cet ACN en 3 h sur le lit catalytique.

L'analyse polarographique de l'ACN traité indique une valeur d'IPOL de 380 mole/t, donc nettement plus élevée qu'avant traitement.

### EXEMPLE 4

Dans un réacteur, on place en lit fixe 140 g d'un catalyseur constitué de Pd et de Ag déposé sur un support d'alumine (0,2 % en poids de Pd, 0,1 % en poids de Ag). Ce catalyseur est commercialisé par la société PROCATALYSE sous la dénomination LD 271

La réaction est réalisée à 30°C et sous une pression d'hydrogène (à chaud) de 5 bar.

L'amino-6 capronitrile mis en oeuvre présente un indice polarographique (IPOL) initial de 92 mole/t. On introduit 299 g de cet ACN en 3 h sur le lit catalytique.

Les résultats obtenus sont rassemblés dans le tableau I ci-dessous.

### EXEMPLE 5

Dans un réacteur, on place en lit fixe 140 g d'un catalyseur constitué de Pd et de Au déposé sur un support d'alumine (0,2 % en poids de Pd et 0,1 % de Au). Ce catalyseur est commercialisé par la société PROCATALYSE sous la dénomination LD 277.

La réaction est réalisée à 30°C et sous une pression d'hydrogène (à chaud) de 5 bar.

L'amino-6 capronitrile mis en oeuvre présente un indice polarographique (IPOL) initial de 92 mole/t. On introduit 299 g de cet ACN en 3 h sur le lit catalytique.

Les résultats obtenus sont rassemblés dans le tableau I ci-dessous.

### EXEMPLE 6

L'exemple 1 est répétée mais avec introduction de monoxyde de carbone dans le flux d'hydrogène en début de réaction. 0,2 mmol de CO sont ainsi introduites, c'est à dire 2,5 mol de CO /T de catalyseur.

L'hydrogénation est réalisée avec 300g de ACN en 3 heures.

Après 3 h l'indice polarographique est de 20 mole/t, l'indice initial étant de 92 mole/t.

### EXEMPLE 7

L'exemple 4 est reproduit mais en utilisant 10 % en poids d'eau comme solvant. La durée de l'hydrogénation est de 3 heures.

| Exemple | Catalyseur | Température en ° C | Pression en bar | Perte en ACN en % poids de ACN engagé | IPOL Départ / Fin |
|---|---|---|---|---|---|
| 1 comparatif | Pd/Al₂O₃ | 80 | 20 | 11 | 77/24 |
| 2 comparatif | Pd/Al₂O₃ | 80 | 20 | 10 | 96/16 |
| 4 | Pd-Ag/Al₂O₃ | 40 | 5 | 0,4 | 92/18 |
| 5 | Pd/Au/Al₂O₃ | 30 | 5 | 1,6 | 92/58 |
| 6 | Catalyseur ex. 1 préconditionné | 80 | 20 | 1,6 | 92/20 |
| 7 | Catalyseur ex.4 avec 10% eau | 40 | 5 | 0,2 | 90/16 |

## Revendications

1. Procédé de purification d'aminonitrile, **caractérisé en ce que** l'on soumet l'aminonitrile à une hydrogénation par l'hydrogène moléculaire, en présence d'un catalyseur contenant au moins un métal choisi parmi le palladium, le platine, le ruthénium, l'osmium, l'iridium, le rhodium, et **en ce que** le dit catalyseur comprend un ou plusieurs éléments promoteurs choisis dans le groupe comportant l'or, l'argent, le cuivre, le chrome, le molybdène, le tungstène, le germanium, l'étain, le plomb, le bore, le gallium, l'indium, le thallium, le phosphore, l'arsenic, l'antimoine, le bismuth, le soufre, le sélénium, le tellure, le manganèse, le rhénium, le vanadium, le titane, le zinc, ou est soumis à une étape de préconditionnement consistant en une mise en contact avec un agent sélectivant avant ou en début de réaction d'hydrogénation.

2. Procédé de purification d'aminonitrile, **caractérisé en ce que** l'on soumet l'aminonitrile à une hydrogénation par l'hydrogène moléculaire, en présence d'un catalyseur contenant au moins un métal choisi parmi le ruthénium, l'osmium, l'iridium, le rhodium.

3. Procédé selon l'une des revendications 1 ou 2, **caractérisé en ce que** l'aminonitrile est l'amino-6-capronitrile.

4. Procédé selon l'une des revendications 1à 3, **caractérisé en ce que** le catalyseur contient au moins du palladium.

5. Procédé selon l'une des revendications 1 à 4, **caractérisé en ce qu'**il est mis en oeuvre à une température comprise entre 20°C et 150°C.

6. Procédé selon l'une des revendications 1 à 5, **caractérisé en ce qu'**il est mis en oeuvre sous une pression absolue d'hydrogène à la température d'hydrogénation située entre 1 bar et 100 bar, et de préférence comprise entre 2 et 50 bar.

7. Procédé selon l'une des revendications 1 à 6, **caractérisé en ce que** le catalyseur mis en oeuvre comprend le métal actif finement divisé et déposé sur un support solide.

8. Procédé selon la revendication 6 ou 7, **caractérisé en ce que** le support du catalyseur est choisi parmi l'alumine, la zircone, le dioxyde de titane, la silice, le charbon actif.

9. Procédé selon l'une des revendications 2 à 8, **caractérisé en ce que** le catalyseur comporte en outre un ou plusieurs éléments promoteurs.

10. Procédé selon l'une des revendications 2 à 9 **caractérisé en ce que** le catalyseur est soumis à une étape de préconditionnement consistant en une mise en contact avec un agent sélectivant avant ou en début de réaction d'hydrogénation.

11. Procédé selon l'une des revendications 1 à 10, **caractérisé en ce que** le rapport pondéral élément promoteur/métal catalyseur se situe entre 0 % et 100 %, de préférence entre 5 et 60%.

12. Procédé selon l'une des revendications précédentes, **caractérisé en ce que** le procédé de préconditionnement du catalyseur consiste à introduire dans le réacteur d'hydrogénation contenant le catalyseur un agent sélectivant en mélange soit avec l'hydrogène soit avec l'aminonitrile à purifier, au début de la réaction d'hydrogénation.

13. Procédé selon l'une des revendications précédentes, **caractérisé en ce que** l'agent sélectivant est choisi dans le groupe comprenant l'oxyde de carbone, les composés organiques soufrés, les composés organiques phosphorés.

14. Procédé selon la revendication 13, **caractérisé en ce que** les composés organiques soufrés sont choisis dans le groupe comprenant la thiourée et ses dérivés, les thiols, les sulfures d'alkyles, les composés organiques phosphorés étant choisis dans le groupe comprenant les phosphites, hypophosphites d'alkyles, phosphates et thiophosphates d'alkyles.

15. Procédé selon l'une des revendications précédentes, **caractérisé en ce que** la quantité d'agent sélectivant ajouté par tonne de catalyseur à préconditionner est comprise entre 0,5 et 15 mole d'agent sélectivant par tonne de catalyseur.

16. Procédé selon l'une des revendications précédentes, **caractérisé en ce que** l'hydrogénation est suivie par une distillation.

## Patentansprüche

1. Verfahren zur Reinigung von Aminonitril, **dadurch gekennzeichnet, dass** man das Aminonitril einer Hydrierung durch molekularen Wasserstoff in Gegenwart eines Katalysators unterzieht, der wenigstens ein Metall enthält, das ausgewählt ist unter Palladium, Platin, Ruthenium, Osmium, Iridium, Rhodium, und dadurch, dass besagter Katalysator ein oder mehrere Promotorelemente umfasst, die ausgewählt sind aus der Gruppe, die Gold, Silber, Kupfer, Chrom, Molybdän, Wolfram, Germanium, Zinn, Blei, Bor, Gallium, Indium, Thallium, Phosphor, Arsen, Antimon, Bismut, Schwefel, Selen, Tellur, Mangan, Rhenium, Vanadium, Titan, Zink umfasst, oder einem Schritt zur Vorkonditionierung unterzogen wird, der aus einem Inkontaktbringen mit einem Selektivierungsmittel vor oder am Anfang der Hydrierungsreaktion besteht.

2. Verfahren zur Reinigung von Aminonitril, **dadurch gekennzeichnet, dass** man das Aminonitril einer Hydrierung durch molekularen Wasserstoff in Gegenwart eines Katalysators unterzieht, der wenigstens ein Metall enthält, das ausgewählt ist unter Ruthenium, Osmium, Iridium, Rhodium.

3. Verfahren gemäß einem der Ansprüche 1 oder 2, **dadurch gekennzeichnet, dass** das Aminonitril 6-Aminocapronitril ist.

4. Verfahren gemäß einem der Ansprüche 1 bis 3, **dadurch gekennzeichnet, dass** der Katalysator wenigstens Palladium enthält

5. Verfahren gemäß einem der Ansprüche 1 bis 4, **dadurch gekennzeichnet, dass** es bei einer Temperatur zwischen 20 °C und 150 °C eingesetzt wird.

6. Verfahren gemäß einem der Ansprüche 1 bis 5, **dadurch gekennzeichnet, dass** es unter einem absoluten Wasserstoffdruck bei der, Hydrierungstemperatur zwischen 1 bar und 100 bar und vorzugsweise zwischen 2 und 50 bar eingesetzt wird.

7. Verfahren gemäß einem der Ansprüche 1 bis 6, **dadurch gekennzeichnet, dass** der eingesetzte Katalysator das aktive Metall fein verteilt und abgeschieden auf einem festen Träger umfasst.

8. Verfahren gemäß Anspruch 6 oder 7, **dadurch gekennzeichnet, dass** der Träger des Katalysators ausgewählt ist unter Aluminiumoxid, Zirkonoxid, Titandioxid, Siliciumdioxid, Aktivkohle.

9. Verfahren gemäß einem der Ansprüche 2 bis 8, **dadurch gekennzeichnet, dass** der Katalysator außerdem ein oder mehrere Promotorelemente umfasst.

10. Verfahren gemäß einem der Ansprüche 2 bis 9, **dadurch gekennzeichnet, dass** der Katalysator einem Schritt zur Vorkonditionierung unterzogen wird, der aus einem Inkontaktbringen mit einem Selektivierungsmittel vor oder am Anfang der Hydrierungsreaktion besteht.

11. Verfahren gemäß einem der Ansprüche 1 bis 10, **dadurch gekennzeichnet, dass** das Gewichtsverhältnis Promotorelement / Katalysatormetall zwischen 0 % und 100 %, vorzugsweise zwischen 5 und 60 % liegt.

12. Verfahren gemäß einem der vorhergehenden Ansprüche, **dadurch gekennzeichnet, dass** das Verfahren zur Vorkonditionierung des Katalysators darin besteht, in den Hydrierungsreaktor, der den Katalysator enthält, ein Selektivierungsmittel im Gemisch entweder mit dem Wasserstoff oder mit dem zu reinigenden Aminonitril am Anfang der Hydrierungsreaktion einzuführen.

13. Verfahren gemäß einem der vorhergehenden Ansprüche, **dadurch gekennzeichnet, dass** das Selektivierungsmittel ausgewählt ist aus der Gruppe, die Kohlenmonoxid, die organischen Schwefelverbindungen, die organischen Phosphorverbindungen umfasst.

14. Verfahren gemäß Anspruch 13, **dadurch gekennzeichnet, dass** die organischen Schwefelverbindungen ausgewählt sind aus der Gruppe, die Thiohamstoff und seine Derivate, die Thiole, die Alkylsulfide umfasst, wobei die organischen Phosphorverbindungen ausgewählt sind aus der Gruppe, die die Alkylphosphite, -hypophosphite, Alkylphosphate und -thiophosphate umfasst.

15. Verfahren gemäß einem der vorhergehenden Ansprüche, **dadurch gekennzeichnet, dass** die Menge an zugefügtem Selektivierungsmittel pro Tonne Katalysator, die vorkonditioniert werden soll, zwischen 0,5 und 15 mol Selektivierungsmittel pro Tonne Katalysator liegt

16. Verfahren gemäß einem der vorhergehenden Ansprüche, **dadurch gekennzeichnet, dass** der Hydrierung eine Destillation folgt.

## Claims

1. Process for the purification of aminonitrile, **characterized in that** the aminonitrile is subjected to a hydrogenation with molecular hydrogen in the presence of a catalyst comprising at least one metal chosen from palladium, platinum, ruthenium, osmium, iridium or rhodium and **in that** the said catalyst comprises one or more promoter elements chosen from the group consisting of gold, silver, copper, chromium, molybdenum, tungsten, germanium, tin, lead, boron, gallium, indium, thallium, phosphorus, arsenic, antimony, bismuth, sulphur, selenium, tellurium, manganese, rhenium, vanadium, titanium and zinc or is subjected to a preconditioning stage which consists in bringing it into contact with a selectivating agent before or at the beginning of the hydrogenation reaction.

2. Process for the purification of aminonitrile, **characterized in that** the aminonitrile is subjected to a hydrogenation with molecular hydrogen in the presence of a catalyst comprising at least one metal chosen from ruthenium, osmium, iridium or rhodium.

3. Process according to either of Claims 1 and 2, **characterized in that** the aminonitrile is 6-aminocapronitrile.

4. Process according to one of Claims 1 to 3, **characterized in that** the catalyst comprises at least palladium.

5. Process according to one of Claims 1 to 4, **characterized in that** it is carried out at a temperature of between 20°C and 150°C.

6. Process according to one of Claims 1 to 5, **characterized in that** it is carried out under an absolute hydrogen pressure at the hydrogenation temperature lying between 1 bar and 100 bar and preferably between 2 and 50 bar.

7. Process according to one of Claims 1 to 6, **characterized in that** the catalyst employed comprises the active metal finely divided and deposited on a solid support.

8. Process according to Claim 6 or 7, **characterized in that** the support of the catalyst is chosen from alumina, zirconia, titanium dioxide, silica or active charcoal.

9. Process according to one of Claims 2 to 8, **characterized in that** the catalyst additionally comprises one or more promoter elements.

10. Process according to one of Claims 2 to 9, **characterized in that** the catalyst is subjected to a preconditioning stage which consists in bringing it into contact with a selectivating agent before or at the beginning of the hydrogenation reaction.

11. Process according to one of Claims 1 to 10, **characterized in that** the promoter element/catalyst metal ratio by weight is between 0% and 100%, preferably between 5 and 60%.

12. Process according to one of the preceding claims, **characterized in that** the process for preconditioning the catalyst consists in introducing into the hydrogenation reactor comprising the catalyst, at the beginning of the hydrogenation reaction, a selectivating agent as a mixture either with the hydrogen or with the aminonitrile to be purified.

13. Process according to one of the preceding claims, **characterized in that** the selectivating agent is chosen from the group comprising carbon monoxide, organic sulphur compounds and organic phosphorus compounds.

14. Process according to Claim 13, **characterized in that** the organic sulphur compounds are chosen from the group comprising thiourea and its derivatives, thiols and alkyl sulphides, the organic phosphorus compounds being chosen from the group comprising alkyl hypophosphites, phosphites, alkyl thiophosphates and phosphates.

15. Process according to one of the preceding claims, **characterized in that** the amount of selectivating agent added per tonne of catalyst to be preconditioned is between 0.5 and 15 mol of selectivating agent per tonne of catalyst.

16. Process according to one of the preceding claims, **characterized in that** the hydrogenation is followed by a distillation.
